# EUROPEAN PATENT APPLICATION

(11) **EP 1 829 525 A1**
(43) Date of publication of application: **05.09.2007**
(21) Application number: 05819909.2
(22) Date of filing: 16.12.2005
(51) Int. Cl.: A61K 8/97, A61K 8/55, A61K 8/44, A61Q 19/08, A61Q 19/00, A61K 36/48, A61K 31/401, A61K 31/6615, A61P 17/00, A61P 43/00

(54) **COMPOSITION AND METHOD FOR ACCELERATING FIBULIN-5 PRODUCTION AND/OR ENHANCING FIBULIN-5 ACTIVITY**

(30) Priority: 17.12.2004 JP 2004365935
(71) Applicant: Shiseido Company, Limited, Chuo-ku Tokyo 104-8010 (JP)
(72) Inventor: OGURA, Yuki, c/o SHISEIDO RESEARCH CENTER, Yokohama-shi, Kanagawa 2368643 (JP); AMANO, Satoshi, c/o SHISEIDO RESEARCH CENTER, Yokohama-shi, Kanagawa 2368643 (JP); SONEHARA, Nobuko, c/o SHISEIDO RESEARCH CENTER, Yokohama-shi, Kanagawa 2368643 (JP)
(74) Representative: Ulmann, Catherine Claire
(86) International application number: PCT/JP2005/023553
(87) International publication number: WO 2006/064975

(57) **Abstract**

There is provided a composition for promoting the production of and/or enhancing the activity of fibulin-5, said composition comprising as an active ingredient one or a plurality of pharmaceutical agents selected from the group consisting of extracts of *Caesalpinia crista,* extracts of *Psophocarpus tetragonolobus,* phytic acid and a pharmaceutically acceptable salt thereof, and L-hydroxyproline and a pharmaceutically acceptable salt thereof.

## Description

### FIELD OF THE INVENTION

The present invention relates to a composition and method for promoting the production of and/or enhancing the activity of fibulin-5.

### BACKGROUND OF THE INVENTION

In organs having stretchability such as the skin, blood vessels and the lung, elasticity is important in order to exhibit their functions. Elastic fibers are extracellular matrices that provide the elastic behavior of tissues, and extensive research is underway on the assembly of elastic fibers from the viewpoint of aging and various physiological phenomena.

Fibrillin-1 shapes the backbone of elastic fiber assembly by forming microfibrils. Elastin is crosslinked and polymerized by the action of lysyl oxidase in the microfibrils and deposits in the tissue to assemble elastic fibers. Fibulins are widely expressed secretory proteins and biologically active substances found in the blood, and in the basement membranes and stroma of most tissues, where they self-associate (Balbona, K. et al., J. Biol. Chem. 267:20120-20125, 1992: Pan, T.C. et al., J. Biol. Chem. 123:1269-1277, 1993), and/or interact with a variety of extracellular matrix components (for example, fibronectin, laminin, nidogen, aggrecan, versican, endostatin, and elastin) (Aspberg, A. et al., J. Biol. Chem. 274:20444-20449, 1999; Nakamura, T. et al., Nature 415:171-175, 2002; Timpl, R. et al., 2003, supra; Yanagisawa, H. et al., Nature 415:168-171, 2002). Fibulins likely participate in the assembly and stabilization of extracellular matrix structures and have also been implicated in regulating organogenesis, vasculogenesis, fibrogenesis, and tumorigenesis (Roark, E.F. et al., J. Histochem. Cytochem. 43:401-411, 1995; Tran, H. et al., J. Biochem. 270:19458-19464, 1995: Zhang, H.Y. et al., Dev. Dyn. 205:348-364, 1996). For the fibulin gene family, five distinct genes are reported, from which at least nine protein products are produced via alternative splicing (Timpl, R. et al., Nat. Rev. Mol. Cell Biol. 4:479-489, 2003).

Key proteins in the elastic fiber assembly remain to be seen. However, the knock-out mice for fibulin-5, the latest member of the fibulin family, exhibited abnormal elastic fiber formation (Nakamura T. et al., 2002, supra; Yanagisawa H. et al., 2002, supra), which revealed that fibulin-5 is important for the assembly of elastic fibers.

Fibulin-5 is a glycoprotein composed of 448 amino acids and has an interesting characteristic structure. Fibulin-5 contains an integrin-binding RGD motif, six calcium-binding EGF-like repeats, a Pro-rich insert in the first calcium-binding EGF-like repeat, and a globular C-terminal domain (Kowal, R.C. et al., Circ. Res. 84:1166-76, 1999; Nakamura, T. et al., 1999, supra). Functionally, fibulin-5 binds αvβ3, αvβ5, and α9β1 integrins (Nakamura, T. et al., 2002, supra) and adheres to endothelial cells via its RGD motif (Nakamura, T. et al., 1999, supra). Inactivation of the fibulin-5 gene in mice produces profound elastinopathy in the skin, lung, and vasculature (Nakamura, T. et al., 2002, supra; Yanagisawa, H. et al., 2002, supra).

The discovery that transforming growth factor beta 1 (TGFb1) that enhances fibulin-5 production (Schiemann W.P. et al., J. Biol. Chem. 2002 Jul. 26, 277(30):27367-77) promotes the reassembly of elastic fibers in the three-dimensional culture system using smooth muscle cells or fibroblasts also revealed that the enhanced production of fibulin-5 is important not only in the assembly of elastic fibers during the embryonal period but also in the reassembly of elastic fibers (Long J.L. et al., Matrix Biol. 2003 Jun. 22(4):339-50). Thus, since the regulation of the production and/or activity of fibulin-5 is important for the regulation of elastic fiber reassembly, substances that can regulate the production and/or activity of fibulin-5 were being sought after.

### DISCLOSURE OF THE INVENTION

It is an object of the present invention to provide a pharmaceutical agent that promotes the production and/or enhances the activity of fibulin-5.

After applying various pharmaceutical agents to human fibroblasts to examine the expression behavior of the fibulin-5 gene, the present inventor has found that particular pharmaceutical agents, specifically extracts of *Caesalpinia crista*, extracts of *Psophocarpus tetragonolobus*, phytic acid and L-hydroxyproline significantly enhance the expression of the fibulin-5 gene, and thereby has completed the present invention.

Thus, the present invention provides the following inventions:
(1) A composition for promoting the production of and/or enhancing the activity of fibulin-5, said composition comprising as an active ingredient one or a plurality of pharmaceutical agents selected from the group consisting of extracts of *Caesalpinia crista,* extracts of *Psophocarpus tetragonolobus*, phytic acid and a pharmaceutically acceptable salt thereof, and L-hydroxyproline and a pharmaceutically acceptable salt thereof;
(2) A method for promoting the production of and/or enhancing the activity of fibulin-5 in a biological tissue by applying to said tissue a composition comprising as an active ingredient one or a plurality of pharmaceutical agents selected from the group consisting of extracts of *Caesalpinia crista,* extracts of *Psophocarpus tetragonolobus,* phytic acid and a pharmaceutically acceptable salt thereof, and L-hydroxyproline and a pharmaceutically acceptable salt thereof;
(3) The method according to (2) wherein said biological tissue is the skin.

In accordance with the present invention, a composition for promoting the production of and/or enhancing the activity of fibulin-5 is provided. Such a composition and a method can be useful in the promotion of the development and reassembly of elastic fibers, aging prevention, wound healing, angiogenesis inhibition and the like.

### BEST MODE FOR CARRYING OUT THE INVENTION

As described at the beginning, fibulin-5 contributes the development and reassembly of normal elastic fibers, and thus the production of fibulin-5 and the expression of its biological activities in biological organs such as the skin, blood vessels and the lung in which elastic fibers play important roles are thought to be essential for the normal function of these organs. Thus, pharmaceutical agents of the present invention (hereinafter referred to simply as "pharmaceutical agents of the present invention") that are effective for promoting the production of and/or enhancing the activity of fibulin-5 are thought to be useful for improving various pathological conditions derived from elastic fiber abnormalities including, for example, emphysema, tortuous arteries, and senile changes in the skin due to aging such as sagged skins and wrinkles.

Furthermore, considering the known functions of fibulin-5, other effects exhibited by the pharmaceutical agents of the present invention include the following:

M.J. Lee et al., J. Am. Coll. Surg. 2004 (Sep.) 199(3):403-10 reported that when a retrovirus containing fibulin-5 was injected into a wounded site of the skin to effect the overexpression of fibulin-5 at the site, the closure of the wound occurred early and healing was promoted. Thus, by applying a pharmaceutical agent of the present invention to a wounded site, it is expected that wound healing can be promoted.

Ping-Ping Kuang et al., Am. J. Physiol. Lung Cell Mol. Physiol. 285(5):L1147-52, Epub 2003 (Aug 8) describes that an increase in the amount of fibulin-5 expressed occurs during the repair of lung disorders in mice with a lung disorder due to hyperoxemia, and thus fibulin-5 is a key protein that heals lung disorders in a concerted action with elastin. Thus, by applying a pharmaceutical agent of the present invention to lung disorders due to hyperoxemia, it is expected that the healing of lung disorders can be promoted.

A.R. Albig et al., DNA Cell Biol. 2004 Jun. 23(6):367-79 describes that fibulin-5 has an ability of inhibiting angiogenesis, and suggests the possibility that it may be used as a novel antagonist for inappropriate angiogenesis. Therefore, pharmaceutical agents of the present invention are expected to have the effect as an antagonist for angiogenesis, for example as a preventive and therapeutic agent for diabetes mellitus, retinopathy, arthritis and cancer.

A composition of the present invention for promoting the production of and/or enhancing the activity of fibulin-5 contains as an active ingredient one or a plurality of pharmaceutical agents selected from the group consisting of extracts of *Caesalpinia crista,* extracts of *Psophocarpus tetragonolobus,* phytic acid and a pharmaceutically acceptable salt thereof, and L-hydroxyproline and a pharmaceutically acceptable salt thereof, and preferably it may further contain pharmaceutically or cosmetically acceptable carriers and/or additives depending on the intended use of said composition.

*"Caesalpinia crista"* is a plant of the family *Leguminosae,* the genus *Caesalpinia. Caesalpinia crista* extracts of the present invention are extracted mainly from the seeds. Such extracts may be prepared in a standard method or may be commercially available products (such as one available from MARUZEN PHARMACEUTICALS CO., LTD.).

*"Psophocarpus tetragonolobus"* is a plant of the family *Leguminosae,* the genus *Psophocarpus. Psophocarpus tetragonolobus* extracts of the present invention are extracted mainly from the seeds. Such extracts may be prepared in a standard method.

The above extracts of *Caesalpinia crista* and *Psophocarpus tetragonolobus* may be produced, as described above, by extracting from respective source plants by commonly known methods. For example, a source plant is ground as it is raw or after being dried as needed, immersed or heated to reflux in an extraction solvent, and then filtered and concentrated to obtain the extract. The extraction solvent used at this time may be any solvent that is used for extraction of plant extracts. For example, there can be mentioned hot water, organic solvent such as lower alcohols (methanol, ethanol, isopropyl alcohol, n-butanol etc.), polyhydric alcohols (propylene glycol, 1,3-butylene glycol etc.), hydrates of these alcohols, hydrocarbon solvents (n-hexane, toluene etc.), chloroform, dichloroethane, carbon tetrachloride, ethyl acetate ester, ether and the like, or mixtures thereof, and preferably ethanol or 1,3-butylene glycol.

The amount mixed of the above extract in a composition of the present invention is 0.0002 - 20.0% by weight, preferably 0.001 - 1.0% by weight as a dry product per total amount of the composition. If the amount is less than 0.0002% by weight, the effect of the present invention cannot be fully exhibited, and if it exceeds 20.0% by weight, the preparation of pharmaceutical formulations is difficult and thus it is not desirable.

Phytic acid, also called "myo-inositol hexakis-phosphate", abundantly occurs in seed plants such as cereals and seedlings, and provides a major reserve for phosphates. It has a potent metal-chelating activity, and can remove or inactivate metal ions such as iron. In recent years, its anti-cancer effect is drawing an attention.

As pharmaceutically acceptable salt forms thereof, there can be mentioned, but not limited to, alkali metal salts such as sodium salts, potassium salts, for example disodium salts and dipotassium salts, alkaline earth metal salts such as calcium salts, magnesium salts, aluminum salts, magnesium salts and calcium-magnesium mixed salts, amine salts formed from various organic amines, i.e. aliphatic or arylaliphatic primary, secondary or tertiary mono-, di- or polyamines, or heterocyclic groups, for example salts derived from triethylamine, 2-hydroxyethylamine, di-(2-hydroxyethyl)amine, tri-(2-hydroxyethyl)amine, 4-aminobenzoic acid-2-dietylaminoethyl ester, 1-ethylpiperidine, bicyclohexylamine, N,N'-dibenzylethylenediamine, pyridine, collidine, quinoline, procaine, dibenzylamine, 1-efenamine and N-alkyl-piperidine. The structure of phytic acid is shown below.

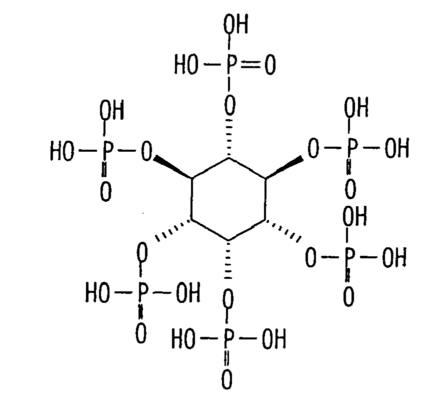

L-hydroxyproline, also called "(2S,4R)-4-hydroxypyrrolidine-2-carboxylic acid", is an imino acid that constitutes structural proteins of animals such as collagen and elastin. As its actions, the promotion of collagen production in fibroblasts, the promotion of epidermal cell growth, a moisture retention effect equal or superior to collagen and the like are known.

As pharmaceutically acceptable salt forms thereof, there can be mentioned, but not limited to, alkali metal salts such as sodium salts, potassium salts, alkaline earth metal salts such as calcium salts, magnesium salts and aluminum salts; amine salts formed from various organic amines, i.e. aliphatic or arylaliphatic primary, secondary or tertiary mono-, di- or polyamines, or heterocyclic groups, for example salts derived from triethylamine, 2-hydroxyethylamine, di-(2-hydroxyethyl)amine, tri-(2-hydroxyethyl)amine, 4-aminobenzoic acid-2-dietylaminoethyl ester, 1-ethylpiperidine, bicyclohexylamine, N,N'-dibenzylethylenediamine, pyridine, collidine, quinoline, procaine, dibenzylamine, 1-efenamine and N-alkyl-piperidine. The structure of L-hydroxyproline is shown below.

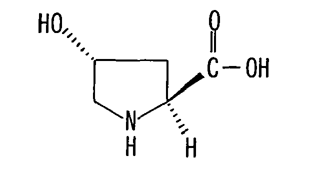

The amount mixed of the above phytic acid or L-hydroxyproline in the composition of the present invention is 0.0002 - 20.0% by weight, preferably 0.001-1.0% by weight per total amount of the composition. If the amount is less than 0.0002% by weight, the effect of the present invention cannot be fully exhibited, and if it exceeds 20.0% by weight, the preparation of pharmaceutical formulations is difficult and thus it is not desirable.

The composition of the present invention may take various dosage forms depending on the intended use thereof. For example, when the composition of the present invention is used as an anti-aging agent for improving the sagging of the skin, it may take any of dosage forms of external preparations for the skin, for example, as cosmetics, liniments such as lotions, essences, gels, foundations, emulsions, creams, ointments, packs, aerosols, gels and liquids, adhesive preparations such as tapes and patches, or nebulae such as sprays and dusts. When the composition of the present invention is used in treatment methods such as prevention and/or treatment of blood vessels and the lung, it may take any of dosage forms such as solutions, suspensions, solids, semi-solids, dusts, granules, capsules and liposome encapsulants suitable for any of the administration methods such as percutaneous, oral, parenteral, enteral, intravenous, subcutaneous and bone marrow injections depending on the treatment methods.

The composition of the present invention may contain any carriers that are compatible with the pharmaceutical agent of the present invention and that are pharmaceutically and cosmetically acceptable according to the purpose and in suitable amounts. Suitable carriers may be for example water, physiological saline, for example phosphate buffered saline, glucose, glycerol, ethanol or mixtures thereof. As desired, furthermore, the composition may contain, as appropriate, adjuvants that augment the effect of the pharmaceutical agent of the present invention, for example pharmaceutically and cosmetically acceptable additives such as wetting agents, humectants, surfactants, emulsifiers, disinfectants, antioxidants, perfumes, flavoring agents and pH buffering agents.

Examples of such pharmaceutically and cosmetically acceptable additives include mannitol, sorbitol, lactose, collagen, polyvinyl alcohol, polyvinyl pyrrolidone, carboxyvinyl polymer, carboxymethyl cellulose sodium, sodium polyacrylate, sodium alginate, water-soluble dextran, carboxymethyl starch sodium, pectin, methylcellulose, ethylcellulose, polyethylene glycol, diglycerin, glycerin, propylene glycol, polyethylene glycol, vaseline, paraffin, stearyl alcohol, stearic acid, human serum albumin (HSA), xanthan gum, gum arabic, casein, gelatin and agar.

For the composition of the present invention, the effect of the prevention of aging, the promotion of wound healing, the promotion of treatment of lung disorders due to hyperoxemia, prevention and treatment of, for example, diabetes mellitus, retinopathy, arthritis and cancer by the inhibition of angiogenesis can be expected. Thus, by applying the composition of the present invention to biological tissues such as the skin tissue, the lung tissue and the blood vessel tissue of mammals including humans, depending on the purpose thereof, the desired effect may be expected. The method of application may be varied depending on the tissue to be applied or the purpose of application, and include, for example, percutaneous, oral, parenteral, enteral, intravenous, subcutaneous and bone marrow injections, and the method and the dosage may be determined by medical experts such as physicians, veterinarians and pharmacists.

The present invention will now be explained below with reference to specific examples. It should be noted, however, that the present invention is not limited to these examples in any way.

### EXAMPLES

### Method of expressing and determining fibulin-5 mRNA

### (1) Culture of human fibroblasts

Human fibroblasts were isolated from the foreskin of a neonate, and cultured in a Dulbecco's Modified Eagle's Medium (DMEM) supplemented with 10% by weight fetal bovine serum (FBS). Adhering cells were suspended by a trypsin-ethylenediaminetetraacetic acid (EDTA) treatment and then filtered to obtain a homogeneous cell suspension. Cells were collected by centrifugation and suspended at 0.2×10⁶ cells/ml. Three ml each of the cell suspension was inoculated to a 6-well plate. After the cells adhered, the medium was replaced with a 0.25% by weight FBS-containing DMEM. After culturing at 37°C for 24 hours, the medium was replaced with 0.25% by weight FBS-containing DMEM containing either of a few dozen of pharmaceutical agents. After culturing for further 24 hours, the culture supernatant was removed, and mRNA was recovered by the ABI PRISM 6100 Nucleic Acid PrepStation Kit (Applied Biosystems).

### (2) Determination of fibulin-5 mRNA by a quantitative PCR method

The amount expressed of fibulin-5 mRNA was analyzed with a quantitative PCR method, the ABI PRISM (Trademark) 7900HT Sequence Detection System (Applied Biosystems), that employs a 5' nuclease assay. The primers and probes used in this Example are as follows:
Upstream primer: 5'-CATCTTGTACCGGGACATGGA-3'
Downstream primer: 5'-CGTTTGCCGCATGTAAAATTCT-3'
Probe: 5'-CAAATGCAAGCCACGACCCGCTACCCT-3'
The determination of mRNA expression was conducted in three groups, and was expressed in percentage relative to the mean of the no-addition control group.

The result, as shown in the table below, revealed that extracts of *Caesalpinia crista* (MARUZEN PHARMACEUTICALS CO., LTD.), extracts of *Psophocarpus tetragonolobus* (90% ethanol), phytic acid (Sigma) and L-hydroxyproline (NIKKO CHEMICALS CO., LTD.) exhibit a significant effect of promoting fibulin-5 production.

**Table 1: Effect of various agents on fibulin-5 production in human fibroblasts**

| Name of agent | Concentration % by weight in () | (% of the control) Mean ± SD |
|---|---|---|
| *Caesalpinia crista* | 5 µg/ml (0.0005%) | 121.1±19.9 |
| | 50 µg/ml (0.005%) | **152.7±44.1** |
| *Psophocarpus tetragonolobus* | 5 µg/ml (0.0005%) | 109.9±27.7 |
| | 50 µg/ml (0.005%) | **153.5±13.3** |
| phytic acid | 25 µg/ml (0.0025%) | 93.0±29.4 |
| | 250 µg/ml (0.025%) | **126.1±16.4** |
| L-hydroxyproline | 25 µg/ml (0.0025%) | 110.1±11.0 |
| | 250 µg/ml (0.025%) | **147.3±8.9** |
| Bold-faced numerals indicate significant difference (P<0.05). | | |

### Prescription Examples

A prescription example of a lotion containing an active ingredient of the present invention that exhibits the effect of anti-aging and wound healing is shown. Prescription example 1: Vanishing cream (O/W type, combined use of soap + a nonionic surfactant)

| | | |
|---|---|---|
| Agent: | *Caesalpinia crista* | 0.1% by weight |
| Oil: | Stearic acid | 8.0 |
| | Stearyl alcohol | 4.0 |
| | Butyl stearate | 6.0 |
| Humectant: | Propylene glycol | 5.0 |
| Surfactant: | Monostearyl glycerol | 2.0 |
| Alkali: | Potassium hydroxide | 0.4 |
| Disinfectant: | | q.s. |
| Antioxidant: | | q.s. |
| Perfume: | | q.s. |
| Purified water: | | 74.5 |

### Manufacture

The pharmaceutical agent, the humectant and the alkali are added to purified water to prepare an aqueous phase, which is then heated to 70°C. After the oil components were heated to melt, the surfactant, the disinfectant, the antioxidant and the perfume are added thereto, which is adjusted to 70°C. The above aqueous phase is added thereto and the mixture is preemulsified. After the emulsified particles are homogenized with a homomixer, it is degassed, filtered and cooled. Prescription example 2: Emollient cream (O/W type, combined use of a nonionic surfactant)

| | | |
|---|---|---|
| Agent: | *Psophocarpus tetragonolobus* | 0.1% by weight |
| Oil: | Stearic alcohol | 6.0 |
| | Stearic acid | 2.0 |
| | Hydrogenated lanolin | 4.0 |
| | Squalane | 9.0 |
| | Octyl dodecanol | 10.0 |
| Humectant: | 1,3-butylene glycol | 6.0 |
| | PEG1500 | 4.0 |
| Surfactant: | POE(25) Cetyl alcohol ether | 3.0 |
| | Monostearyl glycerol | 2.0 |
| Disinfectant: | | q.s. |
| Antioxidant: | | q.s. |
| Perfume: | | q.s. |
| Purified water: | | 53.9 |

### Manufacture

The pharmaceutical agent and the humectant are added to purified water to prepare an aqueous phase, which is then heated to 70°C. After the oil components were heated to melt, the surfactant, the disinfectant, the antioxidant and the perfume are added thereto, which is adjusted to 70°C. The above aqueous phase is added thereto and the mixture is homogenized with a homomixer, which is degassed, filtered and cooled. Prescription example 3: Emollient cream (O/W type, combined use of soap + a nonionic surfactant)

| | | |
|---|---|---|
| Agent: | Phytic acid dipotassium salt | 0.1% by weight |
| Oil: | Cetyl alcohol | 5.0 |
| | Stearic acid | 3.0 |
| | Vaseline | 5.0 |
| | Squalane | 10.0 |
| | Glycerol tri-2-ethyl hexanoic acid ester | 7.0 |
| Humectant: | Dipropylene glycol | 5.0 |
| | Glycerin | 5.0 |
| Surfactant: | Propylene glycol monostearic acid ester | 3.0 |
| | POE(25) Cetyl alcohol ether | 3.0 |
| Alkali: | Triethanolamine | 1.0 |
| Disinfectant: | | q.s. |
| Antioxidant: | | q.s. |
| Perfume: | | q.s. |
| Purified water: | | 52.9 |

### Manufacture

The pharmaceutical agent, the humectant and the alkali are added to purified water to prepare an aqueous phase, which is then heated to 70°C. After the oil components were heated to melt, the surfactant, the disinfectant, the antioxidant and the perfume are added thereto, which is adjusted to 70°C. The above aqueous phase is added thereto and the mixture is preemulsified. After the emulsified particles are homogenized with a homomixer, it is degassed, filtered and cooled. Prescription example 4: Massage cream (O/W type, combined use of beeswax + borax + a nonionic surfactant)

| | | |
|---|---|---|
| Agent: | L-hydroxyproline | 0.1% by weight |
| Oil: | Solid paraffin | 5.0 |
| | Beeswax | 10.0 |
| | Vaseline | 15.0 |
| | Liquid paraffin | 41.0 |
| Humectant: | 1,3-butylene glycol | 4.0 |
| Surfactant: | Monostearic acid glycerin | 2.0 |
| | POE(25) sorbitan monolauric acid ester | 2.0 |
| Alkali: | Borax | 0.2 |
| Disinfectant: | | q.s. |
| Antioxidant: | | q.s. |
| Perfume: | | q.s. |
| Purified water: | | 20.7 |

### Manufacture

The pharmaceutical agent, the humectant and borax are added to purified water to prepare an aqueous phase, which is then heated to 70°C. After the oil components were heated to melt, the surfactant, the disinfectant, the antioxidant and the perfume are added thereto, which is adjusted to 70°C. The above aqueous phase is gradually added thereto and the mixture is preemulsified. After the emulsified particles are homogenized with a homomixer, it is degassed, filtered and cooled.

## Claims

1. A composition for promoting the production of and/or enhancing the activity of fibulin-5, said composition comprising as an active ingredient one or a plurality of pharmaceutical agents selected from the group consisting of extracts of *Caesalpinia crista,* extracts of *Psophocarpus tetragonolobus,* phytic acid and a pharmaceutically acceptable salt thereof, and L-hydroxyproline and a pharmaceutically acceptable salt thereof;

2. A method for promoting the production of and/or enhancing the activity of fibulin-5 in a biological tissue by applying to said tissue a composition comprising as an active ingredient one or a plurality of pharmaceutical agents selected from the group consisting of extracts of *Caesalpinia crista,* extracts of *Psophocarpus tetragonolobus,* phytic acid and a pharmaceutically acceptable salt thereof, and L-hydroxyproline and a pharmaceutically acceptable salt thereof;

3. The method according to claim 2 wherein said biological tissue is the skin.


SEQUENCE LISTING
<110> SHISEIDO CO. LTD.
<120> Composition and Method for Promoting Production and/or Enhancing Act
ivity of Fibulin-5
<130> R921
<150> JP 2004-365935
<151> 2004-12-17
<160> 3

<210> 1
<211> 21
<212> DNA
<213> Artificial Sequence
<223> Forward Primer

<400> 1
catcttgtac cgggacatgg a 21

<210> 2
<211> 22
<212> DNA
<213> Artificial Sequence
<223> Reverse Primer

<400> 2
cgtttgccgc atgtaaaatt ct 22

<210> 3
<211> 27
<212> DNA
<213> Artificial Sequence
<223> Probe

<400> 3
caaatgcaag ccacgacccg ctaccct 27